# EUROPEAN PATENT APPLICATION

(11) **EP 1 375 669 A1**
(43) Date of publication of application: **02.01.2004**
(21) Application number: 02077325.5
(22) Date of filing: 13.06.2002
(51) Int. Cl.: C12N 15/82, A01H 5/00

(54) **Method for enhancing the disease resistance in plants by altering trehalose-6-phosphate levels**

(71) Applicant: STICHTING VOOR DE TECHNISCHE WETENSCHAPPEN, 3527 JP Utrecht (NL)
(72) Inventor: Schlüpmann, Henriette, 2801 NA Gouda (NL); Smeekens, Josephus Christianus Maria, 3971 AE Driebergen (NL)
(74) Representative: Van Someren, Petronella F. H. M.

(57) **Abstract**

The present invention relates to methods for enhancing the disease resistance in plants, especially broad spectrum disease resistance by alteration of trehalose-6-phosphate levels. Said plants are, for example, obtainable by recombinant DNA technology or conventional breeding methods and exhibit enhanced disease resistance against diseases stemming from one or more microorganisms of viral, bacterial, fungal and other origin, especially from one or more microorganisms belonging to the phylum Oomycetes as for example Peronospora parasitica, Phytophtora infestans, Peronospora tabacina, Perespona destructor, Plasmopara viticola, Bremia lactucae.

## Description

The present invention relates to methods for enhancing the disease resistance in plants, especially broad spectrum disease resistance. Said plants are, for example, obtainable by recombinant DNA technology or conventional breeding methods and exhibit enhanced disease resistance against diseases stemming from one or more microorganisms of viral, bacterial, fungal and other origin, especially from one or more microorganisms belonging to the phylum Oomycetes for example Peronospora parasitica, Phytophtora infestans, Peronospora tabacina, Perespona destructor, Plasmopara viticola, Bremia lactucae. The invention further relates to the disease resistant plants obtainable by these methods and the use of said plants in agriculture, forestry and aquaculture.

Disease resistance is economically important in industrialized agriculture. Every year, diseases caused by plant pests are responsible for extensive damage to the industrialized agriculture, which is particularly vulnerable due to the large fields of genetically homogenous monocultures, which allow for rapid spreading of the pests.

Various strategies have been developed to control diseases in plants, yet the most common one generally used is applying large amounts of chemicals, like fungicides, to the infected fields. The used chemicals are often toxic to humans, animals and other plants. In addition, it is difficult to restrain these chemicals to the infected field and they often tend to accumulate in ground and surface waters or in the food chain.

Another approach to control plant diseases is the identification of naturally occurring or induced genotypes which exhibit enhanced disease resistance. However, this approach is tedious and extremely time-consuming. The lack of appropriate germplasm and the incompatibility of crosses limits the application of this method even further.

Yet another approach is the "gene-for-gene resistance" approach by using recombinant DNA technology. The term "gene-for-gene" denotes the dependance of this resistance on a matching specificity between a plant disease resistance gene and a specific pathogen gene. The process is reminiscent of mammalian antibody-antigen interactions. A strong defense response mediated by the immune system of the plant is triggered by the pathogen. The response includes synthesis of antimicrobial enzymes and metabolites, generation of signaling molecules that activate defense in neighboring cells, and reinforcement of plant cell walls surrounding the site of infection.

However, such responses are usually highly dependent on triggering by one specific pathogen. Subsequently, to acquire resistance against more than one pathogens it is necessary to introduce multiple resistance genes into the plant.

Disease resistance against multiple pathogens (broad spectrum resistance) can be obtained by direct triggering of the plant's immune response pathways without the intermediate step involving the pathogen. For example, the expression of NIM1 leads to constitutive activation of Systemically Acquired Resistance (SAR). SAR can also be triggered by an oxidative burst caused by reactive oxygen species or nitric oxide.

SAR is not the only immune response in plants. Others have been described, such as the Induced Systemic Response (ISR), responses to wounding, which generally involve systemin and jasmonic acid, and PAD2 mediated immunity. None, except PAD2, mediate resistance to Phytophtora, the most destructive fungal pest. PAD2 mediated resistance has not yet been commercially exploited.

Members of the phylum Oomycetes, like Peronospora parasitica, Phytophtora infestans, Peronospora tabacina, Perespona destructor, or Plasmopara viticola, can cause complete destruction of important food crops such as potato (late blight, responsible for the Great Famine in Ireland), tomato (late blight), and grape (downy mildew). Until now, no plants with broad spectrum disease resistance, which are also resistant to for example Peronospora parasitica, Phytophtora infestans, Peronospora tabacina, Perespona destructor, or Plasmopara viticola have been reported.

The inventors contemplated that the identification of other mechanisms that trigger alternative pathways of the plants's immune system could yield another type of broad spectrum disease resistance in plants, which includes resistance against diseases stemming from one or more microorganisms of the phylum Oomycetes like Peronospora parasitica, Phytophtora infestans, Peronospora tabacina, Perespona destructor, or Plasmopara viticola.

It is therefore the object of the present invention to provide methods for obtaining plants with enhanced disease resistance, especially plants with broad spectrum disease resistance by alternatively triggering the immune response.

It is also an object of the present invention to provide methods for obtaining plants with enhanced disease resistance, especially plants with broad spectrum disease resistance, against diseases stemming from one or more pests of viral, bacterial or fungal origin.

Another object of the present invention is to provide methods for obtaining plants with enhanced disease resistance, especially plants with broad spectrum disease resistance, which, in addition, are resistant against diseases stemming from one or more pests belonging to the phylum Oomycetes.

Yet another object of the present invention is to provide methods for obtaining plants with enhanced disease resistance, especially plants with broad spectrum disease resistance, which are resistant against diseases stemming from Peronospora parasitica, Phytophtora infestans, Peronospora tabacina, Perespona destructor, or Plasmopara viticola.

Still an other object of the present invention is to provide plants with enhanced disease resistance, especially plants with broad spectrum disease resistance. Said plants exhibit enhanced disease resistance against diseases stemming from one or more microorganisms of viral, bacterial and fungal origin, especially from one or more microorganisms belonging to the phylum Oomycetes like Peronospora parasitica, Phytophtora infestans, Peronospora tabacina, Perespona destructor, or Plasmopara viticola.

Finally, it is an object of the present invention to provide a use of plants with enhanced disease resistance, especially plants with broad spectrum disease resistance, in agriculture, forestry or aquaculture.

Said plants exhibit enhanced disease resistance, i.e. broad spectrum disease resistance, against diseases stemming from one or more microorganisms of viral, bacterial and fungal origin, especially from one or more microorganisms belonging to the phylum Oomycetes for example Peronospora parasitica, Phytophtora infestans, Peronospora tabacina, Perespona destructor, or Plasmopara viticola.

Surprisingly, it was found by the inventors that all the above-mentioned objects of the present invention can be achieved by altering the levels of trehalose-6-phosphate in plants. This can be achieved, for example, by recombinant DNA technology or conventional breeding methods.

Trehalose-6-phosphate is one of the metabolites of the metabolic trehalose pathway found in many organisms.

Trehalose-6-phosphate is usually synthesized by conversion of glucose-6-phosphate and UDP-glucose into trehalose-6-phosphate and UDP by the activity of the enzyme trehalose-6-phosphate synthase (TPS). Trehalose-6-phosphate can be converted into glucose and glucose-6-phosphate and trehalose and inorganic phosphate by the activity of trehalose-6-phosphate hydrolase (TPH)and trehalose-6-phosphate phosphatase, respectively.

The invention is based on the finding that alterations (i.e. either an increase or a decrease) in trehalose-6-phosphate levels in plants lead to enhanced disease resistance, especially broad spectrum resistance. The resistance can be either a direct or an indirect result of the alterations in trehalose-6-phosphate levels. An example of an indirect result is when the trigger of the resistance is a product from a reaction that requires trehalose-6-phosphate. When the trigger is trehalose-6-phosphate itself, the resistance is said to be the direct result of alterations in the trehalose-6-phosphate levels. Both types fall within this invention.

The trehalose-6-phosphate levels in the plant are altered according to the invention by manipulation of the expression levels of one or more genes which influence trehalose-6-phosphate levels directly or through one or more intermediate steps.

The term "manipulation of expression levels" as used herein includes silencing of genes and increasing or decreasing expression levels leading to altered levels of trehalose-6-phosphate.

Manipulation of expression levels can involve genes that can affect trehalose-6-phosphate levels through their encoded protein. Examples of such proteins are enzymes like proteases and kinases, regulatory proteins, signalling proteins such as receptors or proteins involved in signalling cascades, proteins influencing mRNA stability and DNA-binding proteins.

The genes can be either "native" in origin or heterologous. Any heterologous gene whose encoded protein retains its function regarding influencing trehalose-6-phosphate levels in plants can be used. According to one aspect of the present invention, the OtsA gene of E.coli or modified or truncated versions thereof is used. The OtsA gene encodes for a protein which has a similar function in plants as the "native" trehalose-6-phosphate synthase (TPS) enzyme.

The term "truncated or modified versions thereof" as used herein comprises any gene, coding for a protein which still retains, although manipulated or altered, its activity to influence trehalose-6-phosphate levels.

According to one aspect of the present invention, the expression level of genes which are involved in metabolizing trehalose is manipulated. Examples of such genes are the trehalose-6-phosphate synthase (TPS) gene, the trehalose-6-phosphate phosphatase (TPP) gene or the trehalose-6-phosphate hydrolase (TPH) gene or modified or truncated versions thereof.

According to the present invention, the manipulation of expression can be achieved by a method using a DNA construct for transformation of a plant cell, which DNA construct comprises,
a) a gene, or mutated or truncated versions thereof, encoding a protein influencing trehalose-6-phosphate levels;
b) a promoter; and
c) optionally a polyadenylation signal.

The gene can be any gene whose encoded protein directly or indirectly influences trehalose-6-phosphate levels but is preferably chosen from the group consisting of the trehalose-6-phosphate synthase (TPS) gene, the trehalose-6-phosphate phosphatase (TPP) gene or the trehalose-6-phosphate hydrolase (TPH) gene or modified or truncated versions thereof.

The trehalose-6-phosphate gene can be of prokaryotic or eukaryotic origin and is preferably the OtsA gene of E.coli or modified or truncated versions thereof.

Manipulation of expression can also be achieved by influencing RNA levels. An example of such influence is binding of the encoded anti-sense RNA to mRNA coding for proteins influencing trehalose-6-phosphate levels, thereby inhibiting translation of the mRNA. This inhibition leads to a subsequent change in trehalose-6-phosphate levels.

According to the present invention, this type of manipulation can be achieved by a method using a DNA construct for transformation of a plant cell, which DNA construct comprises,
a) a DNA sequence encoding an anti-sense RNA which is anti-sense of a mRNA encoding a protein which influences trehalose-6-phosphate levels,
b) a promoter.

In a preferred embodiment of the present invention, the DNA sequence encodes an RNA in the anti-sense orientation of the mRNAs encoding the proteins trehalose-6-phosphate phosphatase (TPP) or trehalose-6-phosphate hydrolase (TPH)

In addition, expression levels can be manipulated by silencing. Silencing of genes is based on homologous recombination of a DNA with the gene of interest. The DNA is inserted in the targeted gene by means of homologous sequences at the end of the DNA. The insertion site of the DNA is chosen in such a way that the coding sequence of the gene is disrupted. After insertion of the DNA sequence, the disrupted gene can no longer encode a functional protein and the gene is said to be silenced.

This manipulation can be achieved by a method using a DNA construct for transforming a plant cell, which DNA construct comprises a DNA containing at both ends, sequences which are homologous to sequences in a gene encoding a protein influencing trehalose-6-phosphate levels.

Preferred genes to be silenced are the "native" trehalose-6-phosphate phosphatase (TPP) gene or the trehalose-6-phosphate hydrolase (TPH) gene.

The promoter used to drive gene expression or RNA synthesis can be a strong constitutive promoter, for example the CaMV 35S promoter, the Arabidopsis plastocyanin promoter and the rubisco small subunit promoter. Upon introduction of the DNA construct comprising these promoters, expression occurs in all the organs of plant, detectable by altered levels of trehalose-6-phosphate.

These plants exhibit an enhanced broad spectrum resistance, especially against diseases stemming from one or more microorganisms of viral, bacterial, fungal or other origin, such as one or more microorganisms belonging to the phylum Oomycetes.

The promoter can also be an inducible promoter like for example the Arabidopsis thaliana PR1 promoter, the phenyl alanine promoter (PAL) and the chalcone synthase promoter.

The Arabidopsis thaliana PR1 promoter may be induced by pathogen attack or chemical treatment such as benzothiadiazole (BTH) treatment. Once induced, this promoter yields very high levels of expression. The use of induced expression allows the plant to invest its resources in growth and productivity rather than in defense under normal conditions. Upon transformation, regeneration and induction, expression occurs in all the organs of a plant, detectable by altered levels of trehalose-6-phosphate. These plants exhibit an enhanced resistance to diseases of viral, bacterial, fungal or other origin, especially against disease stemming from Oomycetes origin.

The promoter can also be a tissue specific inducible or constitutive promoter, like the papatin promoter of Solanum tuberosum which is used for expression of genes in potato tubers. The use of a tissue specific inducible or constitutive promoter further allows the plant to allocate more resources for growth and productivity rather than for defense under normal conditions.

Upon transformation, and optionally induction, expression occurs in specific organs of the plant, detectable by altered levels of trehalose-6-phosphate. These plants exhibit an enhanced resistance to diseases of viral, bacterial, fungal or other origin, specifically against disease stemming from Oomycetes origin.

The embodiments of the present invention regarding the different promoters can also be used in combination with other methods like methods that lead to SAR-activation.

After transformation of the plant cells the cells are i.e. generated into plants using methods generally known to the person skilled in the art.

The genes or encoded proteins, either directly or indirectly influencing trehalose-6-phosphate levels, can also be used as marker in conventional breeding methods, wherein plants, occurring naturally or obtained by treatment for example with a mutagenic agent, with altered trehalose-6-phosphate levels are selected.

The markers are analyzed by methods generally known to the person skilled in the art such as Southern blotting, Western blotting, Northern blotting or PCR.

After obtaining the plant either by recombinant DNA technology or conventional breeding methods the plants are further propagated by sexual or asexual reproduction using methods generally known to the person skilled in the art.

According to the invention any plant can acquire enhanced disease resistance by alteration of its trehalose-6-phosphate levels. The invention is in particular useful for plants such as Arabidopsis thaliana, mais (Zea mays L.), wheat (Triticum aestiyum L.), barley (Hordeum vulgare), rice (Oryza sativa L.), soya bean (Phaseolus vulgaris L.), sugar beet (Beta vulgaris L.), chicory (Cichorium intybus L.), potato (Solanum tuberosum), rape seed (Brassica napus L.), sugar cane (Saccharum officinarum L.), sweet potato (Diocorea esculenta L.), cassava (Manihot esculenta L.), Lolium spp., Poa spp., and Festuca spp..

The plants obtainable by the methods of the present invention exhibit an increased broad spectrum disease resistance and therefor have an advantage over the plants available according to the present art in agriculture, forestry and aquaculture.

The plants obtainable by the methods according to the invention can be further crossed with plants having other desirable traits, like dwarfism. Dwarfism contributed to the significant increase in rice crop production seen in the 1960s and 1970s, especially in Asia.It is also important for other cereals such as wheat. The trait provides rice cultivars with short, thick culms, raises the harvest index, and responsiveness to nitrogen fertilizer, resulting in high yields without affecting panicle and grain quality.

The invention will be further illustrated in the examples that follow and which are not given to limit the invention. In the examples, reference is made to the following figures:
**Figure 1**: Expression vector used for the overexpression of the E.coli OTSA gene under control of the CaMV 35S promoter.
**Figure 2**: Plants overexpressing the E.coli OTSA gene, 6 days after infection with Peronospora parasitica versus wildtype.

### EXAMPLES

### Example 1. Construction of an expression vector for overexpression of a trehalose-6-phosphate gene in plants.

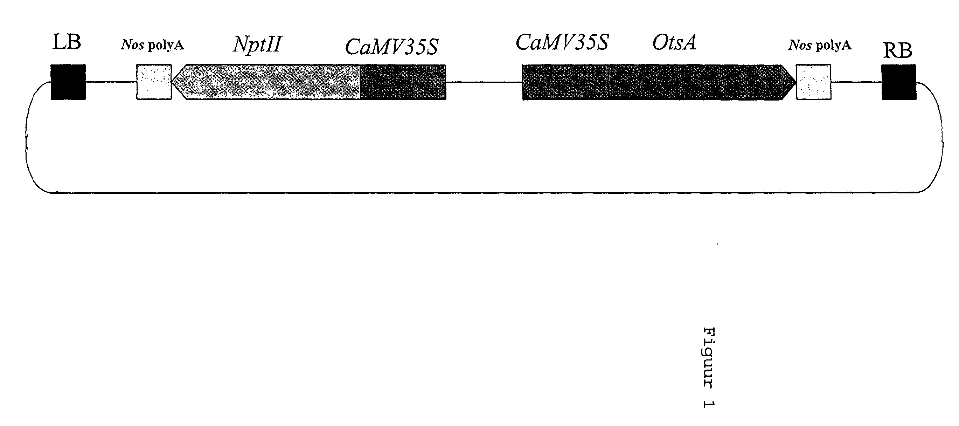

E.coli genomic DNA containing the OtsA gene was prepared from a lysate of lambda clone 7F11 (Kohara et al. 1987, Cell 50:495-508). The complete OtsA coding sequence was then amplified by PCR using the following primers: GAG AAA ATA CCC GGG GTG ATG AC annealing at the 5' and introducing a SmaI restriction site just before the ATG-start and GAT AAT CGT GGA TCC AGA TCC AGA TAA TGT C annealing at the 3' end. The synthetic adapter DNA duplex, AGC TGG CGT GAG GAG CGG TTA ATA AGC TTG AGC T, was ligated to the 3' end of the OtsA PCR fragment introducing a HindIII site. The SmaI/HindIII complete OtsA fragment was then ligated into the SmaI and HindIII digested binary vector pMOG747 described previously in Godijn et al. 1997, Plant Physiology 113:181-190. The CaMV35S/OtsA/NospolyA cassette was then transferred as an EcoRI/SstI fragment into EcoRI/SstI digested pMOG402 resulting in the vector schematically presented in Fig.1.

The vector obtained allows for transfer and incorporation into plant genome of the DNA between left and right border of the T-DNA. This DNA comprises 2 expression cassettes. One expression cassette contains a selectable marker and leads to constitutive expression of the neomycine phosphotransferase that confers resistance of the plants to kanamycin. The other cassette contains E.coli TPS OtsA and leads to constitutive high expression of this enzyme and accumulation of its product trehalose-6-phosphate in plant tissues.

### Example 2. Tranformation of plants with the binary vector.

The binary plasmid from Example 1 was transferred into Agrobacterium tumefaciens strain GV3202. An Agrobacterium clone containing this vector was grown at 28 °C over night in 500 ml Luria broth (LB) supplemented with 50 mg/l kanamycin and 15 mg/l rifampicin. The suspension was then used to transform 4 week old bolting Arabidopsis Col.0 using the floral dip technique described in Clough and Bent 1998, The Plant Journal 16(6):735-743. Transgenic seedlings were selected on agar solidified half strength MS medium supplemented with 25 mg/l kanamycin.

To identify transgenic lines with T-DNA insertion at a single locus, segregation of T2 seedlings was analysed and lines with a ratio of 1:3 sensitive to resistant were selected for further work.

Segregation of the resistance was also scored in T3 generation seedlings to identify T3 seed stock from T2 plants homozygous for the transgene. Presence of the OtsA transgene in the lines obtained was confirmed by Southern blot. Stability of OtsA expression and phenotype were tested until the T5 generation. Plants from lines with stable high levels of expression were tested for their characteristics.

### Example 3. Infection of trehalose-6-phosphate synthase overexpressors with Peronospora parasitica.

Seed from wt and transgenic lines were sown, stratified 3 days at 4°C, then grown under long day conditions at 22°C for 7 days. Peronospora parasitica spores were collected from infected wt seedlings 8 days after infection by clipping leaves and suspending these in water. Collection was until a spore concentration of 50 spores per micro liter water was obtained. The spore suspension, or just water as a control, was sprayed onto the test seedlings, the seedlings were allowed to dry for 5 min and were then transferred to sealed containers to achieve growth under saturating humidity, long days and 22 °C. After 6 days leaves were clipped and stained using the lactophenol trypan blue protocol as described by Keogh et al. 1980, Trans. Br. Mycol. Soc. 74:329-333.

Leaves from seedlings sprayed with water did not stain whether it be wt leaves or leaves from OtsA over-expressing plants. OtsA over-expressing plants are hence not constitutively induced in the hypersensitive response.

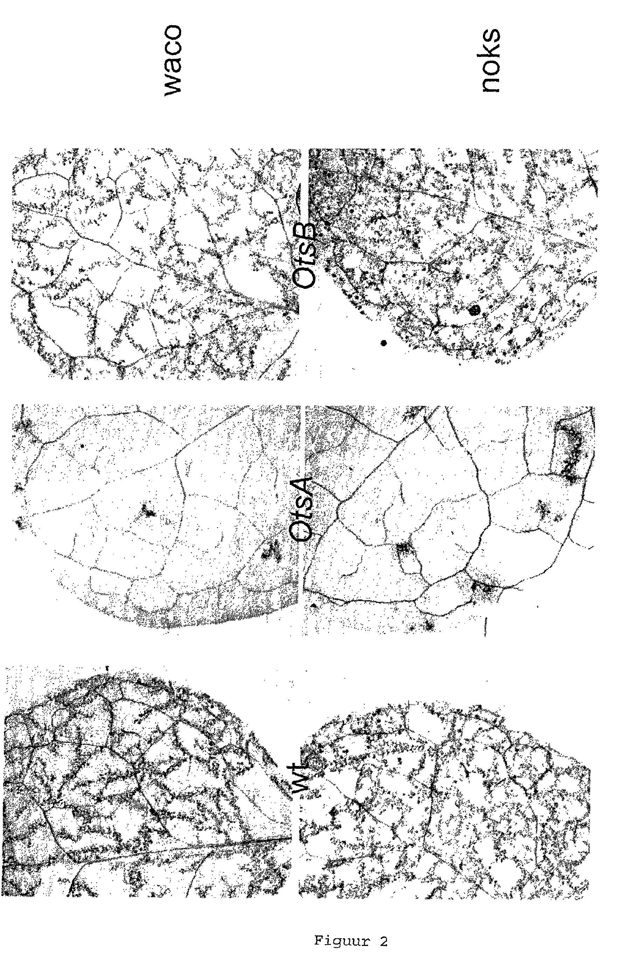

Typical results obtained from leaves of seedlings sprayed with *Peronospora* pathovar wako or noks are shown in Fig.2. Staining shows infection trails over the entire surface of wt leaves whereas none of these are found in leaves of plants that over-express OtsA.

Also shown, are typical results obtained with leaves from plants that over-express the trehalose phosphate phosphatase from *E.coli,* OtsB. Expression of this gene leads to conversion of trehalose-6-phosphate to trehalose and hence possible accumulation of trehalose. Expression of OTSB does not lead to resistance and shows that levels of trehalose-6-phosphate are important in this resistance mechanism rather than trehalose.

## Claims

1. Method for enhancing disease resistance in plants by altering trehalose-6-phosphate levels in the plant.

2. Method according to claim 1, wherein trehalose-6-phosphate levels are altered by manipulation of expression of one or more genes whose encoded protein influences trehalose-6-phosphate levels.

3. Method according to claims 1 or 2, wherein the gene is chosen from the group consisting of the trehalose-6-phosphate synthase (TPS) gene, the trehalose-6-phosphate phosphatase (TPP) gene or the trehalose-6-phosphate hydrolase (TPH) gene or modified or truncated versions thereof.

4. Method according to claims 1 or 2, wherein trehalose-6-phosphate levels are increased by overexpression of one or more genes encoding protein(s), which mediate the conversion of trehalose into trehalose-6-phosphate or the conversion of Glc6-phosphate and UDPG1c to trehalose-6-phosphate.

5. Method according to claim 4, wherein the gene is the trehalose-6-phosphate synthase (TPS) gene or modified or truncated versions thereof.

6. Method according to any of the claims 1-5, wherein the enhanced disease resistance is broad spectrum disease resistance.

7. Method according to claims 1-6, wherein the disease resistance is resistance against one or more diseases stemming from one or more microorganisms of viral, bacterial, fungal origin.

8. Method according to claims 1-7, wherein disease resistance is resistance against one or more diseases stemming from organisms of the phylum Oomycetes.

9. Method according to claims 1-8, wherein the microorganisms are chosen from the group consisting of Peronospora parasitica, Phytophtora infestans, Peronospora tabacina, Perespona destructor, Plasmopara viticola, Bremia lactucae.

10. Method according to any of the claims 1-9, wherein the plants are chosen from the group consisting of Arabidopsis thaliana, mais (Zea mays L.), wheat (Triticum aestiyum L.), barley (Hordeum vulgare), rice (Oryza sativa L.), soya bean (Phaseolus vulgaris L.), sugar beet (Beta vulgaris L.), chicory (Cichorium intybus L.), potato (Solanum tuberosum), rape seed (Brassica napus L.), sugar cane (Saccharum officinarum L.), sweet potato (Diocorea esculenta L.), cassava (Manihot esculenta L.), Lolium spp., Poa spp., Festuca spp.

11. Method according to any of the claims 1-10, wherein the plants are obtained by using a DNA construct for transformation of a plant cell, which DNA construct comprises,
a) a gene, or mutated or truncated versions thereof, encoding a protein influencing trehalose-6-phosphate levels;
b) a promoter; and optionally
c) a polyadenylation signal.

12. Method according claim 11, wherein the gene is chosen from the group consisting of the trehalose-6-phosphate synthase (TPS) gene, the trehalose-6-phosphate phosphatase (TPP) gene or the trehalose-6-phosphate hydrolase (TPH) gene or modified or truncated versions thereof.

13. Method according to any of the claims 1-10, wherein the plants are obtained by transforming into a plant cell a DNA construct, which DNA construct comprises,
a) a DNA sequence influencing in antisense orientation trehalose-6-phosphate levels; and
b) a promoter.

14. Method according claim 13, wherein the DNA sequence is derived from the genes trehalose-6-phosphate phosphatase (TPP) or trehalose-6-phosphate hydrolase (TPH).

15. Method according to any of the claims 1-10, wherein the plants are obtained by using a DNA construct for transforming a plant cell, which DNA construct comprises, a DNA containing at both ends sequences which are homologous to sequences in a gene encoding a protein influencing trehalose-6-phosphate levels.

16. Method according claim 15, wherein the gene is the trehalose-6-phosphate phosphatase (TPP) gene or the trehalose-6-phosphate hydrolase (TPH) gene or mutated or truncated versions thereof.

17. Method according any of the claims 11-16, wherein the promoter is a constitutive promoter.

18. Method according to claim 17, wherein the constitutive promoter is chosen from the group of promoters consisting of the CaMV 35S promoter, the Arabidopsis plastocyanin promoter and the rubisco small subunit promoter.

19. Method according to any of the claims 11-16, wherein the promoter is an inducible promoter.

20. Method according to claim 19, wherein the inducible promoter is chosen from the group of promoters consisting of the PR1 promoter, the phenyl alanine promoter (PAL) and the chalcone synthase promoter.

21. Method according to any of the claims 11-16, wherein the promoter is a tissue specific inducible or constitutive promoter.

22. Method according to claim 21, wherein the inducible promoter is the papatin promoter.

23. Method according to any of the claims 1-22, wherein the trehalose-6-phosphate synthase (TPS) gene is the OtsA gene of E.coli or modified or truncated versions thereof.

24. Method according to any of the claims 1-23, wherein the plant cell is regenerated into a plant.

25. Method according to any of the claims 1-10, wherein the plant is obtained by using conventional breeding methods by selection of genetic variations in plants occurring naturally or by treatment using as marker a gene or protein which influences trehalose-6-phosphate levels.

26. Method according to claim 25, wherein the marker is chosen from the group consisting of the trehalose-6-phosphate synthase gene (TPS) or the trehalose-6-phosphate phosphatase (TPP) gene or trehalose-6-phosphate hydrolase (TPH) or mutated or truncated versions thereof.

27. Method according to claim 1-26, wherein the plant obtained is further propagated by sexual or asexual reproduction.

28. Method according claim 1-27, wherein the plant is further crossed with other plants having other desirable traits.

29. Method according to claims 1-28, wherein the plant is further crossed with other plants with the trait dwarfism.

30. Plant with increased broad spectrum disease resistance obtainable by any of the methods of claims 1-29.

31. Use of the plant obtainable by any of the methods of claim 1-29 in agriculture, forestry and aquaculture.
